# EUROPEAN PATENT SPECIFICATION

(11) **EP 1 699 475 B1**
(45) Date of publication and mention of the grant of the patent: **09.05.2012**
(21) Application number: 04791038.5
(22) Date of filing: 29.10.2004
(51) Int. Cl.: A61K 8/97, A61Q 19/08, A61K 36/79, A61Q 5/00, A61P 17/00

(54) **COSMETIC USE OF AN EXTRACT OF THE FRUIT OF SCHISANDRA CHINENSIS**
KOSMETISCHE VERWENDUNG EINES EXTRAKTES AUS DER FRUCHT VON SCHISANDRA CHINENSIS
UTILISATION COSMÉTIQUE D'UN EXTRAIT DE FRUIT DE SCHISANDRA CHINENSIS

(30) Priority: 07.11.2003 EP 03292802
(43) Date of publication of application: 13.09.2006
(73) Proprietor: BASF Beauty Care Solutions S.A.S., 69007 Lyon (FR)
(72) Inventor: HENRY, Florence, F-54600 Villers-les-Nancy (FR); DANOUX, Louis, F-54420 Saulxures Les Nancy (FR); PAULY, Gilles, F-54000 Nancy (FR)
(74) Representative: Reinhardt, Jürgen
(86) International application number: PCT/EP2004/012278
(87) International publication number: WO 2005/044289

(56) References cited:
- EP-A1- 1 537 873
- EP-A1- 1 658 879
- WO-A-00/28959
- WO-A-01/41778
- WO-A-97/16155
- CN-A- 1 099 971
- CN-A- 1 352 902
- JP-A- 2000 178 168
- JP-A- 20032 861 179
- KR-A- 20020 044 266
- KR-A- 20030 013 695
- US-A- 4 098 882
- DATABASE WPI Section Ch, Week 199444 Derwent Publications Ltd., London, GB; Class B04, AN 1994-354614 XP002339698 & JP 06 279256 A (CLUB COSMETICS KK) 4 October 1994 (1994-10-04)
- DATABASE WPI Section Ch, Week 200251 Derwent Publications Ltd., London, GB; Class B04, AN 2002-477603 XP002339699 & KR 2002 000 134 A (BYUN H C) 4 January 2002 (2002-01-04)
- DATABASE WPI Section Ch, Week 199906 Derwent Publications Ltd., London, GB; Class B04, AN 1999-064806 XP002339700 & JP 10 310532 A (DAIICHI PHARM CO LTD) 24 November 1998 (1998-11-24)
- DATABASE WPI Section Ch, Week 199834 Derwent Publications Ltd., London, GB; Class B05, AN 1998-393357 XP002339701 & JP 10 158126 A (SHISEIDO CO LTD) 16 June 1998 (1998-06-16)
- DATABASE WPI Section Ch, Week 199710 Derwent Publications Ltd., London, GB; Class B05, AN 1997-103635 XP002339702 & JP 08 337510 A (ADVANCED SKIN RES KENKYUSHO KK) 24 December 1996 (1996-12-24)
- KIM YOUNGLEEM ET AL: "Effect of plant matrix and fluid ethanol concentration on supercritical fluid extraction efficiency of schisandrin derivatives" JOURNAL OF CHROMATOGRAPHIC SCIENCE, vol. 37, no. 12, December 1999 (1999-12), pages 457-461, XP009052016 ISSN: 0021-9665
- HANCKE J L ET AL: "SCHISANDRA CHINENSIS (TURCZ.) BAILL" FITOTERAPIA, IDB HOLDING, MILAN, IT, vol. 70, no. 5, 1999, pages 451-471, XP001180872 ISSN: 0367-326X
- DATABASE WPI Section Ch, Week 198933 Derwent Publications Ltd., London, GB; Class B05, AN 1989-237720 XP002339703 & JP 01 172355 A (TSUMURA & CO) 7 July 1989 (1989-07-07)
- DATABASE WPI Section Ch, Week 200473 Derwent Publications Ltd., London, GB; Class B04, AN 2004-744336 XP002339704 & KR 2004 059 098 A (JIN YANG PHARM CO LTD) 5 July 2004 (2004-07-05)
- CHOI Y H ET AL: 'OPTIMUM SFE CONDITION FOR LIGNANS OF SCHISANDRA CHINENSIS FRUI' CHROMATOGRAPHIA, VIEWEG UND TEUBNER VERLAG vol. 48, no. 9/10, 01 November 1998, DE, pages 695 - 699, XP009052018

## Description

The present invention is concerned with the use of the extract of the fruit of Schlsandra chinensis for the cosmetic treatment of the human body.

Schisandra chinensis means the plant that in detail is called Schisandra chinensis (Turcz.) Baill. "Turcz." and "Balll" denote the nomenclature system used.

Schisandra chinensis (in the following text sometimes abbreviated as Schisandra) belongs to the family Schisandracess. It is a woody creeping vine with numerous clusters of tiny bright red berries that is native to Northern China and the adjacent regions of Russia and Korea. In ancient China, Schisandra was used as a staple food for hunting and gathering tribes. As a traditional medicinal herb, Schisandra, called Wu-wei-tzu in China, has been used as an astringent for a treatment for dry cough, asthma, night sweats, nocturnal seminal emissions and chronic diarrhoea. It is also used traditionally as a tonic for the treatment of chronic fatigue. Schisandra is also known by the names Magnolia Vine, Japanese Gomishi, Korean Omicha, and Fruit Of Five Flavours. The common name Schisandra (sometimes spelled Schizandra) includes the species Schisandra fructus, which is used interchangeably with Schisandra chinensls.

During the early 1980's, Chinese doctors began researching Schisandra as a possible treatment for hepatitis, based on its potential for liver-protective effects and the nature of its active constituents. As a result of that research, Schisandra is now a recognized "adaptogen", capable of increasing the body's resistance to disease, stress, and other debilitating processes. Known active constituents include sesquicarene, lignans (schizandrin, gomisin), schizoandrol, citral, phytosterols (stigmasterol, beta-sitosterol), and Vitamins C and E.

In Asia, this adaptogenic property is said to "stimulate immune defences, balance body function, normalize body systems, boost recovery after surgery, protect against radiation, counteract the effects of sugar, optimise energy in times of stress, increase stamina, protect against motion sickness, normalize blood sugar and blood pressure, reduce high cholesterol, shield against infection, improve the health of the adrenals, energize RNA-DNA molecules to rebuild cells and produces energy comparable to that of a young athlete."

Studies conducted on Schisandra's effects have revealed that the herb has a stimulating effect in low doses, but this effect disappears with larger doses. The compounds thought responsible for the liver-protective effects of Schisandra are lignans composed of two phenylpropanoids. More than 30 of these have been isolated in Schisandra, and some 22 of which were tested in 1984 by the Japanese scientist H. Hikino for their ability to reduce the cytotoxic effects of carbon tetrachloride and galactosamine on cultured rat liver cells **(**Hikino, H. et al (1984) anti-hepatotoxic actions of lignoids from Schisandra chinensis fruits, Planta Medica Volume 50(3), pages 213 to 218**)**.

Most lignans were found to be effective, and some were extremely active *(schizandrins* A and B, gomisin A, B-bisabolne). Subsequent Japanese studies have found that two of the lignans, wuweizisu C and gomisin A, exert their liver protective effects by functioning as antioxidants to prevent the lipid peroxydation produced by harmful substances such as carbon tetrachloride. Since lipid peroxydation leads to the formation of liver damage, the two compounds did indeed exert a protective influence.

Western herbalists commonly recommend Schisandra as support for the lungs, liver and kidneys, and to help with depression due to adrenergic exhaustion. In Russia, Schisandra is used to treat eye fatigue and increase acuity.

The publication "J. L. Hancke, R. A. Burgos and F. Ahumada, Fitoterapia, 1999, number 70, pages 451 to 471" discloses different aspects of the pharmacology of the fruit of Schisandra chinensis and of dibenzo-[a,c]-cyclooctene lignans from this plant. Antiheapatotoxic, antioxidant and antitumoral activities and effects on the physical performance and on the central nervous system are disclosed.

According to the internet-data-base "Duke data base", which is a phytochemical and ethnobotanical data base, Schisandra contains the following compounds.:
LIGNAN, BENZOYLISOGOMISIN-O, EPIGOMISIN-O, ANGELOYLGOMISIN-O, ANGELOYLGOMISIN-P, ANGELOYLGOMISIN-Q, ANGELOYLISOGOMISIN-O, GAMMA-SCHIZANDRIN, GOMISIN-A to J, K1, L1, M1, M2, N, O, R, PREGOMISIN, SCHISANTHERIN-D, TIGLOYLGOMISIN-P, WUWEIZISU-C, PROTEIN, ASCORBIC-ACID, CARBOHYDRATES, FAT (LINOLENIC ACID, PALMITIC ACID, PALMITOLEIC ACID, STEARIC ACID, STEROIDS, TOCOPHEROLS, FIBER

The following information is available concerning these compounds.

### Information on schizandrin:

Synonyms: 5,6,7,8-Tetrahydro-1,2,3,10,11,12-hexamethoxy-6,7-dimethyldibenzo [a,c]cycloocten-6-ol, 9Cl; 8-Hydroxy-3,3',4,4',5,5'-hexamethoxy-2,2'-cyclolignan; Wuweizichun A; Wuweizi alcohol A; Schisandrin; Schisandrol A; Schizandrol. CAS Registry Number: 7432-28-2, Molecular Formula: C24H32O7, Molecular Weight: 432.513, Biological Use/Importance: Microsomal cytochrome P 450 inducer. Antihepatotoxic agent. Free radical scavenger, RTECS Accession Number: HP1626000.

The RTECS Accession Number gives access to toxicity data from NIOSH Registry of Toxic Effects of Chemical Substances which is a compendium of toxicity data obtained from literature.

### Information on gomisin A:

Synonyms: 3,3',4,5-Tetramethoxy-4',5'-methylenedioxy-2,2'-cyclolignan-8-ol. Wuweizisu B. Wuweizichum B. Schisandrol B. Schizandrol B. Wuweizi alcohol B. Besigomsin. TJN. CAS Registry Number: 58546-54-6, Related CAS Registry Numbers(s): 61281-39-8, Molecular Formula: C23H28O7, Molecular Weight: 416.47, Biological Use/importance: Antineoplastic agent. Hepatoprotective agent, RTECS Accession Number: DE8396900.

### Information on deoxyschizandrin:

Synonymes: 3,3',4,4',5,5'-Hexamethoxy-2,2'-cyclolignan. Deoxyschisandrin. Schizandrin A. Schisandrin A. Wuweizisu A. Dimethylgomisin.

CAS Registry Number: 61281-38-7, Molecular Formula: C24H32O6, Molecular Weight: 418.513, RTECS Accession Number: HP1595000.

### Information on gomisin N:

CAS Registry Number: 89178-52-9, Molecular Formula; C23H28O6, Molecular Weight: 400.471, Use/importance: Shows insecticidal props.

### Informatlon on wuweizisu C:

Synonyms: Schisandrin C. Schizandrin.

CAS Registry Number: 61301-33-5, Molecular Formula: C22H24O6, Molecular Weight: 384.428, Biological Use/Importance: Inhibits ACAT. Chitin synthase II Inhibitor, RTECS Accession Number: GX9500000

The problem underlying the present invention is the need for substances that can be used in cosmetic applications. There is a need for such substances having a regenerating and revitalising effect on human skin.

This problem is solved by the use of the extract of the fruit of Schisandra chinensis according to the claims of the present patent.

The extract of the fruit of Schisandra chinensis is called the extract according to the present invention. The composition as defined in the previous paragraph is called the composition according to the present invention.

One embodiment of the present invention is the use according to the present invention wherein b) is selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, Kim Youngleem et al., Effect of plant matrix and fluid ethanol concentration on supercritical fluid extraction efficiency of schisandrin derivatives, Journal of chromatographic science, vol. 37, no. 12, 1999, pages 457-461 discloses an extraction process from Schisandra chinensis fruits by supercritical CO2 in comparison with organic solvent extraction.

KR 20030013695 discloses anti-wrinkle compositions which prevent aging of the skin. The compositions comprise amongst other extracts from Schisandra fructus.

CN 1099971 discloses a plant essence skin caring powder composed of Chinese herbal medicines such as Fructus Schisandrae chinanals. The product has wrinkle reducing, skin caring, skin luster improving effect.

KR 20020044266 discloses a skin care cosmetic composition containing composite natural drug extract, which has an excellent skin aging-prevention and skin whitening effect. Schlsandra chinensis is enumerated as one of the drug extracts.

JP2000178168 discloses anti-ageing cosmetic compositions which comprise elastase inhibitor containing plant extracts, such as a Schisandra chinensis fruit extract, as active ingredient, alone or in combination with further elastase inhibitor containing plant extracts. The fruits of Schisandra chinensis are extracted with water, alcohols or organic solvents.

According to Choi, Optimum SFE Condition for lignans of Schisandra chinensis fruits, Chromatographia 48:695-99 (1998), extraction of Schisandra chinensis fruits with supercritical CO2 can be used as an alternative to conventional extraction with organic solvents.

CN1352902 refers to an instant tea comprising an extract of schisandra fruit, isomaltoligose, grape syrup, tea polyphenol, maltodextrin and flavoring. It has healthcare function of delaying sanility. An anti-ageing biological activity of a carbon dioxide supercritical fluid of Schisandra chinensis is mentioned, however no cosmetic effect. antidandruff agents, swelling agents, insect repellents, hydrotropes, solubilizers, preservatives, perfume oils and dyes.

The extraction with supercritical carbon dioxide is carried out preferably at a pressure of 100 to 280 bar and at a temperature of 50 to 60 °C.

One embodiment of the present invention is the use according to the present invention wherein the process for the production of the extract according to the present invention is carried out in such a way that the extract contains lignans.

In one embodiment of the present invention the process for the production of the extract according to the present invention further comprises drying of the extract.

The extract according to the present invention can be purified. For example it can be purified by fractionation (e. g. by low pressure chromatography on polymethacrylate or polymeric adsorbents or by reversed phase C18-chromatography). Supercritical fluids can be used for this purification.

The extract according to the present invention has many advantages. It has many biological activities. E. g. it protects against UV-A and UV-B radiation and against IR radiation, it stimulates GAG synthesis (GAG is an abbreviation for glycosaminoglycannes, GAG are macromolecules of polymerised sugars such as glucosamin, GAG are important components of the human dermis), it is astringent, it reduces the frequency of contraction of innerved human muscular cells by neurons, it stimulates the synthesis of proteoglycans such as syndecan and lumican. WO 2004/054532 (internal file no. of the applicant: C 2720) discloses a cosmetic treatment method for improving and/or protecting the human skin using at least one proteoglycan modulating agent.

Cosmetic treatment of the human body according to the present invention comprises the treatment of skin and/or hairs and/or skin appendices. Skin appendices means nails, sebaceous glands, sweat glands etc..

The auxiliaries and additives which are common for cosmetic purposes can be selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

In one embodiment of the present invention the auxiliaries and additives which are common for cosmetic purposes are selected from the group consisting of surfactants, emulsifiers, fats, waxes, stabilizers, deodorants, antiperspirants, antidandruff agents and perfume oils.

The total content of auxiliaries and additives may be 1 to 50% by weight, preferably 5 to 40% by weight, based on the cosmetic and/or pharmaceutical preparations. The preparations can be prepared by customary cold or hot processes; preference is given to using the phase-inversion temperature method.

For the purposes of the invention, cosmetic preparations can mean care agents. Care agents are understood as meaning care agents for skin and hair. These care agents include, inter alia, cleansing and restorative action for skin and hair.

Application can be topical or oral in the form of tablets, dragees, capsules, juices, solutions and granules.

The compositions and cosmetic preparations according to the invention can be used for the preparation of cosmetic and/or dermopharmaceutical preparations, e.g. hair shampoos, hair lotions, foam baths, shower baths, creams, gels, lotions, alcoholic ana aqueous/alcoholic solutions, emulsions, wax/fat compositions, stick preparations, powders or ointments. Furthermore, the preparations for oral application according to the invention can also be incorporated into tablets, dragees, capsules, juices, solutions and granules.

These preparations can also comprise, as further auxiliaries and additives which are common for cosmetic purposes, oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, antidandruff agents, film formers, swelling agents, insect repellents, hydrotropes, solubilizers, preservatives, perfume oils, dyes and other auxiliaries and additives which are common for cosmetic purposes.

Surfactants (or Surface-active substances) that may be present are anionic, non-ionic, cationic and/or amphoteric or amphoteric surfactants, the content of which in the compositions is usually about 1 to 70% by weight, preferably 5 to 50% by weight and in particular 10 to 30% by weight. Typical examples of anionic surfactants are soaps, alkylbenzenesulfonates, alkanesulfonates, olefin sulfonates, alkyl ether sulfonates, glycerol ether sulfonates, α-methyl ester sulfonates, sulfo fatty acids, alkyl sulphates, fatty alcohol ether sulphates, glycerol ether sulphates, fatty acid ether sulphates, hydroxy mixed ether sulphates, monoglyceride (ether) sulphates, fatty acid amide (ether) sulphates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, e. g. acyl lactylates, acyl tartrates, acyl glutamates and acyl aspartates, alkyl oligoglucoside sulphates, protein fatty acid condensates (in particular wheat-based vegetable products) and alkyl (ether) phosphates. If the anionic surfactants contain polyglycol ether chains, these may have a conventional homologous distribution, but preferably have a narrowed homologous distribution. Typical examples of non-ionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers or mixed formals, optionally partially oxidized alk(en)yl oligoglycosides or glucoronic acid derivatives, fatty acid N-alkyglucamides, protein hydrolysates (in particular wheat-based vegetable products), polyol fatty acid esters, sugar esters, sorbitan esters, polysorbates and amine oxides. If the non-ionic surfactants contain polyglycol ether chains, these may have a conventional homologous distribution, but preferably have a narrowed homologous distribution. Typical examples of cationic surfactants are quaternary ammonium compounds, e. g. dimethyldistearyl-ammonium chloride, and ester quats, in particular quaternized fatty acid trialkanolamine ester salts. Typical examples of amphoteric or zwitterionic surfactants are alkylbetaines, alkylamidobetaines, aminopropionates, aminoglycinates, imidazolinium-betaines and sulfobetaines. Said surfactants are known compounds. With regard to structure and preparation of these substances, reference may be made to relevant review works.

Typical examples of particularly suitable mild, i.e. particularly skin-compatible surfactants are fatty alcohol polyglycol ether sulphates, monoglyceride sulphates, mono- and/or dialkyl sulfosuccinates, fatty acid isethionates, fatty acid sarcosinates, fatty acid taurides, fatty acid glutamates, α-olefinsulfonates, ether carboxylic acids, alkyl oligoglucosides, fatty acid glucamides, alkylamidobetaines, amphoacetals and/or protein fatty acid condensates, the latter preferably based on wheat proteins.

Suitable oily bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, for example myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particutar Z-ethylhexanol, esters of C₁₈-C₃₈-alkylhydroxycarboxylic acids with linear or branched C₆-C₂₂-fatty alcohols, in particular dioctyl malates, esters of linear and/or branched fatty acids with polyhydric alcohols (for example propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, for example dicaprylyl carbonates (Cetiol^{®} CC), Guerbet carbonates based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or unsymmetrical dialkyl ethers having 6 to 22 carbon atoms per alkyl group, for example dicaprylyl ether (Cetiol^{®} OE), ring-opening products of epoxidized fatty acid esters with polyols, silicone oils (cyclomethicones, silicon methicone types, inter alia) and/or aliphatic or naphthenic hydrocarbons, for example squalane, squalene or dialkylcyclohexanes.

Suitable emulsifiers are, for example, nonionogenic surfactants from at least one of the following groups:
- addition products of from 2 to 30 mol of ethylene oxide and/or 0 to 5 mol of propylene oxide onto linear fatty alcohols having 8 to 22 carbon atoms, onto fatty acids having 12 to 22 carbon atoms, onto alkylphenols having 8 to 15 carbon atoms in the alkyl group, and onto alkylamines having 8 to 22 carbon atoms in the alkyl radical;
- alkyl and/or alkenyl oligoglycosides having 8 to 22 carbon atoms in the alk(en)yl radical and the ethoxylated analogs thereof;
- addition products of from 1 to 15 mol of ethylene oxide onto castor oil and/or hydrogenated castor oil;
- addition products of from 15 to 60 mol of ethylene oxide onto castor oil and/or hydrogenated castor oil;
- partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide;
- partial esters of polyglycerol (average degree of self-condensation 2 to 8), polyethylene glycol (molecular weight 400 to 5 000), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide;
- mixed esters of pentaerythritol, fatty acids, citric acid and fatty alcohols and/or mixed esters of fatty acids having 6 to 22 carbon atoms, methylglucose and polyols, preferably glycerol or polyglycerol,
- mono-, di- and trialkyl phosphates, and mono-, di- and/or tri-PEG alkyl phosphates and salts thereof;
- wool wax alcohols;
- polysiloxane-polyalkyl-polyether copolymers and corresponding derivatives;
- block copolymers, e.g. polyethylene glycol-30 dipolyhydroxystearates;
- polymer emulsifiers, e.g. Pemulen^{®} grades (TR-1, TR-2) from Goodrich;
- polyalkylene glycols, and
- glycerol carbonate.

The addition products of ethylene oxide and/or of propylene oxide onto fatty alcohols, fatty acids, alkylphenols or onto castor oil are known, commercially available products. These are homologous mixtures whose average degree of alkoxylation corresponds to the ratio of the amounts of ethylene oxide and/or propylene oxide and substrate with which the addition reaction is carried out. C_{12/18}-fatty acid mono- and diesters of addition products of ethylene oxide onto glycerol are known as refatting agents for cosmetic preparations.

Alkyl and/or alkenyl oligoglycosides, their preparation and their use are known from the prior art. They are prepared, in particular, by reacting glucose or oligosaccharides with primary alcohols having 8 to 18 carbon atoms. With regard to me glycoside radical, both monoglycosides, in which a cyclic sugar radical is glycosidically bonded to the fatty alcohol, and also oligomeric glycosides having a degree of oligomerization of up to, preferably, about 8, are suitable. The degree of oligomerization here is a statistical average value that is based on a homologous distribution customary for such technical-grade products.

Typical examples of suitable partial glycerides are hydroxy stearic acid monoglyceride, hydroxy stearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linoleic acid monoglyceride, linoleic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride, and the technical-grade mixtures thereof which may also comprise small amounts of triglyceride as a minor product of the preparation process. Likewise suitable are addition products of 1 to 30 mol, preferably 5 to 10 mol, of ethylene oxide onto said partial glycerides.

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate, and technical-grade mixtures thereof. Likewise suitable are addition products of 1 to 30 mol, preferably 5 to 10 mol, of ethylene oxide onto said sorbitan esters.

Typical examples of suitable polyglycerol esters are polyglyceryl-2 dipolyhydroxystearate (Dehymuls^{®} PGPH), polyglycerol-3 diisostearate (Lameform^{®} TGl), polyglyceryl-4 isostearate (Isolan^{®} GI 34), polyglyceryl-3 oleate, diisostearoyl polyglyceryl-3 diisostearate (Isolan^{®} PDI), polyglyceryl-3 methylglucose distearate (Tego Care^{®}450), polyglyceryl-3 beeswax (Cera Bellina^{®}), polyglyceryl-4 caprate (Polyglycerol Caprate T2010/90), polyglyceryl-3 cetyl ether (Chimexane^{®} NL), polyglyceryl-3 distearate (Cremophor^{®} GS 32) and polyglyceryl polyricinoleate (Admul^{®} WOL 1403), polyglyceryl dimerate isostearate, and mixtures thereof. Examples of further suitable polyol esters are the mono-, di- and triesters, optionally reacted with 1 to 30 mol of ethylene oxide, of trimethylolpropane or pentaerythritol with lauric acid, coconut fatty acid, tallow fatty acid, palmitic acid, stearic acid, oleic acid, behenic acid.

Furthermore, zwitterionic surfactants can be used as emulsifiers. The term "zwitterionic surfactants" refers to those surface-active compounds that carry at least one quaternary ammonium group and at least one carboxylate and one sulfonate group in the molecule. Particularly suitable zwitterionic surfactants are the betaines, such as N-alkyl-N,N-dimethylammonium glycinates, for example cocoalkyldimethylammonium glycinate, N-acylaminopropyl-N, N-dimethylammonium glycinates, for example cocoacylaminopropyl-dimethylammonium glycinate, and 2-alkyl-3-carboxymethyl-3-hydroxyethylimidazolines having in each case 8 to 18 carbon atoms in the alkyl or acyl group, and cocoacylaminoethylhydroxyethylcarboxymethyl glycinate. Particular preference is given to the fatty acid amide derivative known under the CTFA name *Cocamidopropyl Betaine.* Likewise suitable emulsifiers are ampholytic surfactants. The term "ampholytic surfactants" means those surface-active compounds that, apart from a C_{8/18}-alkyl or - acyl group in the molecule, contain at least one free amino group and at least one - COOH or -SO₃H group and are capable of forming internal salts. Examples of suitable ampholytic surfactants are N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids having in each case about 8 to 18 carbon atoms in the alkyl group. Particularly preferred ampholytic surfactants are N-cocoalkyl aminopropionate, cocoacylaminoethyl aminopropionate and C_{12/18}-acylsarcosine. Finally, cationic surfactants are also suitable emulsifiers, those of the ester quat type, preferably methyl-quaternized difatty acid triethanolamine ester salts, being particularly preferred.

Fats and waxes that can be used are described in the following text. Typical examples of fats are glycerides, i.e. solid or liquid vegetable or animal products which consist essentially of mixed glycerol esters of higher fatty acids, suitable waxes are inter alia natural waxes, for example candelilla wax, carnauba wax, japan wax, esparto grass wax, cork wax, guaruma wax, rice germ oil wax, sugarcane wax, ouricury wax, montan wax, beeswax, shellac wax, spermaceti, lanolin (wool wax), uropygial grease, ceresin, ozokerite (earth wax), petrolatum, paraffin waxes, microcrystalline waxes; chemically modified waxes (hard waxes), for example montan ester waxes, sasol waxes, hydrogenated jojoba waxes, and synthetic waxes, for example polyalkylene waxes and polyethylene glycol waxes. In addition to the fats, suitable additives are also fat-like substances, such as lecithins and phospholipids. The term lecithins is understood by the person skilled in the art as meaning those glycerophospholipids which form from fatty acids, glycerol, phosphoric acid and choline by esterification. Lecithins are thus frequently also [lacuna] as phosphatidylcholines (PC). Examples of natural lecithins which may be mentioned are the cephalins, which are also referred to as phosphatidic acids and represent derivatives of 1,2-diacyl-sn-glycerol-3-phosphoric acids. By contrast, phospholipids are usually understood as meaning mono- and, preferably, diesters of phosphoric acid with glycerol (glycerophosphates), which are generally considered to be fats. In addition, sphingosines and sphingolipids are also suitable.

Examples of suitable pearlescent waxes are: alkylene glycol esters, specifically ethylene glycol distearate; fatty acid alkanolamides, specifically coconut fatty acid diethanolamide; partial glycerides, specifically stearic acid monoglyceride; esters of polybasic, optionally hydroxy-substituted carboxylic acids with fatty alcohols having 6 to 22 carbon atoms, specifically long-chain esters of tartaric acid; fatty substances, for example fatty alcohols, fatty ketones, fatty aldehydes, fatty ethers and fatty carbonates, which have a total of at least 24 carbon atoms, specifically laurone and distearyl ether; fatty acids, such as stearic acid, hydroxystearic acid or behenic acid, ring-opening products of olefin epoxides having 12 to 22 carbon atoms with fatty alcohols having 12 to 22 carbon atoms and/or polyols having 2 to 15 carbon atoms and 2 to 10 hydroxyl groups, and mixtures thereof.

Bodying agents and thickeners that can be used are described in the following text. Suitable bodying agents are primarily fatty alcohols or hydroxy fatty alcohols having 12 to 22, and preferably 16 to 18, carbon atoms, and also partial glycerides, fatty acids of hydroxy fatty acids. Preference is given to a combination of these substances with alkyl oligoglucosides and/or fatty acid N-methylglucamides of identical chain length and/or polyglycerol pvly-12-hydroxystearates. Suitable thickeners are, for example, Aerosil grades (hydrophilic silicas), polysaccharides, in particular xanthan gum, guar guar, agar agar, alginates and Tyloses, carboxymethylcellulose and hydroxyethylcellulose, and also relatively high molecular weight polyethylene glycol mono- and diesters of fatty acids, polyacrylates (e.g. Carbopols^{®} and Pemulen grades from Goodrich; Synthalens^{®} from Sigma; Keltrol grades from Kelco; Sepigel grades from Seppic; Salcare grades from Allied Colloids), polyacrylamides, polymers, polyvinyl alcohol and polyvinylpyrrolidone, surfactants, for example ethoxylated fatty acid glycerides, esters of fatty acids with polyols for example pentaerythritol or trimethylolpropane, fatty alcohol ethoxylates having a narrowed homolog distribution or alkyl oligoglucosides, and electrolytes such as sodium chloride and ammonium chloride.

Superfatting agents which can be used are substances for example lanolin and lecithin, and polyethoxylated or acylated lanolin and lecithin derivatives, polyol fatty acid esters, monoglycerides and fatty acid alkanolamides, the latter also serving as foam stabilizers.

Stabilizers which can be used are metal salts of fatty acids, for example magnesium, aluminium and/or zinc stearate or ricinoleate.

Polymers that can be used are described in the following text. Suitable cationic polymers are, for example, cationic cellulose derivatives, for example a quaternized hydroxyethylcellulose obtainable under the name Polymer JR 400^{®} from Amerchol, cationic starch, copolymers of diallylammonium salts and acryl amides, quaternized vinylpyrrolidone-vinylimidazole polymers, for example Luviquat^{®} (BASF), condensation products of polyglycols and amines, quaternized collagen polypeptides, for example lauryldimonium hydroxypropyl hydrolysed collagen (Lamequat^{®}L/Grünau), quaternized wheat polypeptides, polyethyleneimine, cationic silicone polymers, for example amodimethicones, copolymers of adipic acid and dimethylaminohydroxypropyl-diethylenetriamine (Cartaretins^{®}/Sandoz), copolymers of acrylic acid with dimethyl diallylammonium chloride (Merquat^{®}550/Chemviron), polyaminopolyamides and cross linked water-soluble polymers thereof, cationic chitin derivatives, for example quaternized chitosan, optionally in microcrystanine dispersion, condensation products from dihaloalkyls, for example dibromobutane with bisdialkylamines, for example bis-dimethylamino-1,3-propane, cationic guar gum, for example Jaguar^{®} CBS, Jaguar^{®} C-17, Jaguar^{®} C-16 from Celanese, quaternized ammonium salt polymers, for example Mirapol^{®} A-15, Mirapol^{®} AD-1, Mirapol^{®} AZ-1 from Miranol.

Suitable anionic, zwitterionic, amphoteric and nonionic polymers are, for example, vinyl acetate-crotonic acid copolymers, vinylpyrrolidone-vinyl acrylate copolymers, vinyl acetate-butyl maleate-isobornyl acrylate copolymers, methyl vinyl ether-maleic anhydride copolymers and esters thereof, uncrosslinked polyacrylic acids and polyacrylic acids crosslinked with polyols, acrylamidopropyltrimethylammonium chloride-acrylate copolymers, octylacrylamide-methyl methacrylate-tert-butylaminoethyl methacrylate-2-hydroxypropyl methacrylate copolymers, polyvinylpyrrolidone, vinylpyrrolidone-vinyl acetate copolymers, vinylpyrrolidone-dimethylaminoethyl methacrylate-vinylcaprolactam terpolymers, and optionally derivatized cellulose ethers and silicones.

Suitable silicone compounds are, for example, dimethylpolysiloxanes, methylphenylpolysiloxanes, cyclic silicones, and amino-, fatty-acid-, alcohol-, polyether-, epoxy-, fluorine-, glycoside- and/or alkyl-modified silicone compounds, which can either be liquid or in resin form at room temperature. Also suitable are simethicones, which are mixtures of dimethicones having an average chain length of from 200 to 300 dimethylsiloxane units and hydrogenated silicates.

Deodorants and antimicrobial agents that can be used are described in the following text. Cosmetic deodorants counteract, mask or remove body odors. Body odors arise as a result of the effect of skin bacteria on apocrine perspiration, with the formation of degradation products which have an unpleasant odor. Accordingly, deodorants comprise active ingredients which act as antimicrobial agents, enzyme inhibitors, odor absorbers or odor masking agents. Suitable antimicrobial agents are, in principle, all substances effective against gram-positive bacteria, for example 4-hydroxybenzoic acid and its salts and esters, N-(4-chlorophenyl)-N'-(3,4-dichlorophenyl)urea, 2,4,4'-trichloro-2'-hydroxydiphenyl ether (triclosan), 4-chloro-3,5-dimethylphenol, 2,2'-methylenebis(6-bromo-4-chlorophenol), 3-methyl-4-(1-methyfethyl)phenol, 2-benzyl-4-chlorophenol, 3-(4-chlorophenoxy)-1,2-propanediol, 3-iodo-2-propynyl butylcarbamate, chlorohexidine, 3,4,4'-trichlorocarbanilide (TTC), antibacterial fragrances, thymol, tnyme oil, eugenol, oil of cloves, menthol, mint oil, famesol, phenoxyethanol, glycerol monocaprate, glycerol monocaprylate, glycerol monolaurate (GML), diglycerol monocaprate (DMC), salicylic acid N-alkylamides, for example n-octylsalicylamide or n-decylsalicylamide.

Suitable enzyme inhibitors are preferably, for example, esterase inhibitors. These are preferably trialkyl citrates, such as trimethyl citrate, tripropyl citrate, triisopropyl citrate, tributyl citrate and, in particular, triethyl citrate (Hydagen^{®} CAT). The substances inhibit enzyme activity, thereby reducing the formation of odor. Other substances which are suitable esterase inhibitors are sterol sulfates or phosphates, for example lanosterol, cholesterol, campesterol, stigmasterol and sitosterol sulfate or phosphate, dicarboxylic acids and esters thereof, for example glutaric acid, monoethyl glutarate, diethyl glutarate, adipic acid, monoethyl adipate, diethyl adipate, malonic acid and diethyl malonate, hydroxycarboxylic acids and esters thereof, for example citric acid, malic acid, tartaric acid or diethyl tartrate, and zinc glycinate.

Suitable odor absorbers are substances which are able to absorb and largely retain odor-forming compounds. They lower the partial pressure of the individual components, thus also reducing their rate of diffusion. It is important that in this process perfumes must remain unimpaired. Odor absorbers are not effective against bacteria. They comprise, for example, as main constituent, a complex zinc salt of ricinoleic acid or specific, largely odor-neutral fragrances which are known to the person skilled in the art as "fixatives", for example extracts of labdanum or styrax or certain abietic acid derivatives. The odor masking agents are fragrances or perfume oils, which, in addition to their function as odor masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal raw materials, for example civet and castoreum. Typical synthetic fragrance compounds are products of the ester, ether, aldehyde, ketone, alcohol and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Ethereal oils of relatively low volatility, which are mostly used as aroma components, are also suitable as perfume oils, e.g. sage oil, camomile oil, oil of cloves, melissa oil, mint oil, cinnamon leaf oil, linden flower oil, juniper berry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evemyl, iraldein gamma, phenylacetic acid, geranyl acetate, benzyl acetate, rose oxide, romilat, irotyl and floramat alone or in mixtures.

Antiperspirants reduce the formation of perspiration by influencing the activity of the eccrine sweat glands, thus counteracting underarm wetness and body odor. Aqueous or anhydrous formulations of antiperspirants typically comprise one or more of the following ingredients: astringent active ingredients, oil components, nonionic emulsifiers, coemulsifiers, bodying agents, auxiliaries, for example thickeners or complexing agents, and/or nonaqueous solvents, for example ethanol, propylene glycol and/or glycerol.

Suitable astringent antiperspirant active ingredients are primarily salts of aluminum, zirconium or of zinc. Such suitable antihydrotic active ingredients are, for example, aluminum chloride, aluminum chlorohydrate, aluminum dichlorohydrate, aluminum sesquichlorohydrate and complex compounds thereof, e.g. with 1,2-propylene glycol, aluminum hydroxyallantoinate, aluminum chloride tartrate, aluminum zirconium trichlorohydrate, aluminum zirconium tetrachlorohydrate, aluminum zirconium penta-chlorohydrate and complex compounds thereof, e.g. with amino acids, such as glycine. In addition, customary oil-soluble and water-soluble auxiliaries may be present in antiperspirants in relatively small amounts. Such oil-soluble auxiliaries may, for example, be anti-inflammatory, skin-protective or perfumed ethereal oils, synthetic skin-protective active ingredients and/or oil-soluble perfume oils.

Customary water-soluble additives are, for example, preservatives, water-soluble fragrances, pH regulators, e.g. buffer mixtures, water-soluble thickeners, e.g. water-soluble natural or synthetic polymers, for example xanthan gum, hydroxyethylcellulose, polyvinylpyrrolidone or high molecular weight polyethylene oxides.

Film formers that can be used are described in the following text. Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof, and similar compounds.

Suitable antidandruff active ingredients are piroctone olamine (1-hydroxy-4-methyl-6-(2,4,4-trimethy)pentyt)-2-(1H)-pyridinone monoethanolamine salt), Baypival® (climbazole), Ketoconazole^{®}, (4-acetyl-1-{-4-[2-(2,4-dichlorophenyl) r-2-(1H-imidazol-1-ylmethyl)-1,3-dioxylan-c-4-ylmethoxyphenyl}piperazine, ketoconazole, elubiol, selenium disulfide, colloidal sulfur, sulfur polyethylene glycol sorbitan monooleate, sulfur ricinol polyethoxylate, sulfur tar distillates, salicyclic acid (or in combination with hexachlorophene), undecylenic acid monoethanolamide sulfosuccinate Na salt, Lamepon^{®} UD (protein undecylenic acid condensate), zinc pyrithione, aluminum pyrithione and magnesium pyrithione/dipyrithione magnesium sulfate.

The swelling agents for aqueous phases may be montmorillonites, clay mineral substances, Pemulen, and alkyl-modified Carbopol grades (Goodrich).

Suitable insect repellents are N,N-diethyl-m-toluamide, 1,2-pentanediol or ethyl butylacetylaminopropionate.

To improve the flow behavior, hydrotropes, for example ethanol, isopropyl alcohol, or polyols, can be used. Polyols which are suitable here preferably have 2 to 15 carbon atoms and at least two hydroxyl groups. The polyols can also contain further functional groups, in particular amino groups, or be modified with nitrogen. Typical examples are:
- glycerol;
- alkylene glycols, for example, ethylene glycol, diethylene glycol, propylene glycol, butylene glycol, hexylene glycol, and polyethylene glycols with an average molecular weight of from 100 to 1 000 daltons;
- technical-grade oligoglycerol mixtures with a degree of self-condensation of from 1.5 to 10, for example, technical-grade diglycerol mixtures with a diglycerol content of from 40 to 50% by weight;
- methylol compounds, such as trimethylolethane, trimethylolpropane, trimethylolbutane, pentaerythritol and dipentaerythritol;
- lower alkyl glucosides, in particular those with 1 to 8 carbon atoms in the alkyl radical, for example methyl and butyl glucoside;
- sugar alcohols with 5 to 12 carbon atoms, for example sorbitol or mannitol,
- sugars with 5 to 12 carbon atoms, for example glucose or sucrose;
- amino sugars, for example glucamine;
- dialcohol amines, such as diethanolamine or 2-amino-1,3-propanediol.

Suitable preservatives are, for example, phenoxyethanol, formaldehyde solution, parabenes, pentanediol or sorbic acid, and the other classes of substance listed in Annex 6, Part A and B of the Cosmetics Directive.

Perfume oils which may be used are preferably mixtures of natural and synthetic fragrances. Natural fragrances are extracts from flowers (lily, lavender, rose, jasmine, neroli, ylang-ylang), stems and leaves (geranium, patchouli, petitgrain), fruits (aniseed, coriander, cumin, juniper), fruit peels (bergamot, lemon, orange), roots (mace, angelica, celery, cardamom, costus, iris, calmus), woods (pine wood, sandalwood, guaiac wood, cedarwood, rosewood), herbs and grasses (tarragon, lemon grass, sage, thyme), needles and branches (spruce, fir, pine, dwarf-pine), resins and balsams (galbanum, elemi, benzoin, myrrh, olibanum, opoponax). Also suitable are animal raw materials, for example civet and castoreum. Typical synthetic fragrance compounds are products of the ester, ether, aldehyde, ketone, alcohol and hydrocarbon type. Fragrance compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethylmethylphenyl glycinate, allyl cyclohexylpropionate, styrallyl propionate and benzyl salicylate. The ethers include, for example, benzyl ethyl ether, the aldehydes include, for example, the linear alkanals having 8 to 18 carbon atoms, citral, citronellal, citronellyloxyacetaldehyde, cyclamen aldehyde, hydroxycitronellal, lilial and bourgeonal, and the ketones include, for example, the ionones, α-isomethylionone and methyl cedryl ketone, the alcohols include anethole, citronellol, eugenol, isoeugenol, geraniol, linalool, phenylethyl alcohol and terpineol, and the hydrocarbons include predominantly the terpenes and balsams. Preference is, however, given to using mixtures of different fragrances which together produce a pleasing fragrance note. Ethereal oils of relatively low volatility, which are mostly used as aroma components, are also suitable as perfume oils, e.g. sage oil, camomile oil, oil of cloves, melissa oil, mint oil, cinnamon leaf oil, linden blossom oil, juniper berry oil, vetiver oil, olibanum oil, galbanum oil, labolanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Dyes which can be used are the substances which are approved and suitable for cosmetic purposes. These dyes are normally used in concentrations of from 0.001 to 0.1% by weight, based on the total mixture.

### Examples

### Preparation of an ethanol-water-extract ("the extract") Reference example

In the following examples an extract of the fruit of Schisandra chinensis was used (called "the extract"). It was obtained by extracting fruits of Schisandra chinensis with a mixture of 75 % by weight ethanol and 25 % by weight water.
Extract ratio: 5:1 (= yield 20%) (5 g of fruit gave 1 g of extract)

### Preparation of an extract with supercritical carbon dioxide ("the SC-extract")

5 kg of dry fruit of Schisandra chinensis ("raw material") (the moisture content of the raw material was 20.75 % by weight) were extracted using supercritical carbon dioxide. The raw material was placed in a vessel in the presence of the supercritical carbon dioxide. The pressure applied was 130 bar, the temperature 55 °C, the flow rate of the supercritical carbon dioxide was 25 kg/h, 184 kg of supercritical carbon dioxide were necessary to extract 486 g raw material.

### Example 1: protection of human fibroblasts against UV-A radiation

The aim of this test is to evaluate the capacities against oxidative stress of the extract by a test on a cell culture of human fibroblasts in vitro. This test in vitro evaluates the capacities of cytophotoprotection of human fibroblasts against UV-A radiation. UV-A radiation is chosen because it penetrates the epidermis and reaches the dermis (human skin has an upper layer called epidermis supported by a lower layer called dermis) where it induces oxidative stress that is revealed in particular by lipoperoxydation of cytoplasm membranes. The formed lipoperoxydes split in malondialdehyde responsible of the reticulation of many biological molecules such as proteins (inhibition of enzymes) and nucleic bases (mutagenesis).

The fibroblasts were seeded in a defined culture medium with foetal calf serum (FCS).

The extract (or the SC-extract) has been incubated 1 to 2 days at 3/°C and CO2=5% (atmosphere with 5 % by volume of carbon dioxide in air) with cultured fibroblasts, then the culture medium was replaced by a saline solution and the fibroblasts were irradiated by a dose of UV-A (20 J/cm2; tubes MAZDA FLUOR TFWN40; MAZDA FLUOR TFWN40 is a known type of a purchasable sunlamp). At the end of the irradiation, the level of MDA (malondialdehyde) was measured in the supernatant by a chemical reaction of MDA with thiobarbituric acid which produces a coloured compound the concentration of which was determined by measuring optical density and the level of proteins was measured in the cell homogenate by Bradford's method. Bradford's method is a known method to quantify the level of proteins (Bradford MM., A rapid and sensitive method for the quantification of microgram quantities of protein utilizing the principle of protein-dye binding. Anal. Biochem., Vol 72, pages 248 to 254, 1976).

The results are expressed as % versus the irradiated control or MDA, and the nonirradiated control for proteins then as an average value of 2 tests in triplicate:

### Results for the extract:

| | Dose (% w/v) | Released MDA | Cellular Proteins |
|---|---|---|---|
| Control without UV | | 0 | 100 |
| UV-A 20 J/cm² | | 100 | 111 |
| vitamin E + UV-A | 0.0003 | 8 | 110 |
| The extract + UV-A | 0.003 | 69 | 135 |
| | 0.01 | 26 | 143 |

| | | | |
|---|---|---|---|
| % w/v means weight per volume, i. e. 1 % w/v means 1 g per 100 ml. | | | |

The data show that the extract distinctly reduces the rate of released MDA from irradiated fibroblasts without any toxic effect on the cellular protein rate (cellular protein rate meaning the rate of protein included in cultured cells).

### Results for the SC-extract:

| | Dose (% w/v) | Released MDA | Cellular proteins |
|---|---|---|---|
| Control without UV | | 0 | 100 |
| UV-A 20 J/cm² | | 100 | 105 |
| vitamin E + UV-A | 0.0003 | 42 | 96 |
| UV-A + SC-extract | 0.001 | 109 | 182 |
| | 0.003 | 40 | 115 |

### Example 2: inhibition of melanin synthesis; whitening activity

The whitening activity of the extract was evaluated on a cell line of melanocytes (melanocytes B16 were used; this is a cell line known to the skilled person in the art). The melanocytes were exposed to the extract for 3 days at 37 °C and then the melanocytes were homogenised in 1 n NaOH and the level of melanin was evaluated by measuring the optical density at 475 nm. The level of proteins in the cells was determined by Bradford's method.

The results are expressed in % refering of the control and the whitening potential is caracterised by the ratio of melanin level by the protein level (or melanin / protein) for a non toxic concentration of the tested compound.

### Results in % against control (average of 2 assays in triplicate):

| | Dose (% w/v) | Proteins | Melanin | Index = Proteins/Melanin |
|---|---|---|---|---|
| Control | 0 | 100 | 100 | 1.00 |
| | 0.001 | 106 | 93 | 1.14 |
| Extract according to the described process | 0.003 | 109 | 82 | 1.32 |
| | 0.01 | 121 | 46 | 2.62 |
| Kojic acid | 0.03 | 89 | 26 | 3.37 |
| Arbutin | 0.2 | 86 | 54 | 1.60 |

The presented data show that the extract distinctly reduced the rate of melanin synthesised (- 54 % at a concentration of 0,01 %) without any toxic effect on the cellular protein rate.

### Example 3: Survival efficacy tests on human fibroblasts

The purpose of these tests was to evaluate the toxicity and then the regenerati ng and revitalising activities on human fibroblasts cultured in vitro.

Human fibroblasts were inoculated in a standard medium of a cell culture with foetal calf serum (FCS). After an incubation of 3 days the cells became quiescent, then the growth medium was exchanged for a standard medium with a range of concentrations for each ingredient to be tested. After an incubation of 3 days, the number of viable cells was determined by evaluation of the levels of and the rates of cellular DNA (fluorescent probe), ATP (enzymatic method), proteins (Bradford's method) and GSH which is evaluated according to the method of Hissin (Hissin P.J., Hilf R. A, fluorometric method for determination of oxidised and reduced Glutathione in tissues. Analytical Biochemistry (1977) volume 74, pages 214-226).

ATP (or adenosine triphosphate) is a compound rich in energy. It is mainly produced by mitochondria. The cells need ATP for the activity of many enzymes which control the cytoskeleton, the ionic channels, the nutriment intake, and a lot of other biological processes.

Glutathione (GSH) is a peptide produced by cells to protect them from oxidative stress or certain pollutants like Mercury or Lead. The three amino acids involved in the reduced form of GSH are linked by specific cytoplasmic enzymes, which use ATP. An increase in the GSH level enhances the activity of glutathion-S-transferase, a detoxification enzyme.

The following table shows the results of the test: results in % against control (average of 2 assays in triplicat):

| | Dose (% w/v) | DNA | ATP | Proteins | GSH/proteins |
|---|---|---|---|---|---|
| Control | 0 | 100 | 100 | 100 | 100 |
| SC-extract | 0.0003 | 100 | 142 | 126 | 89 |
| | 0.001 | 140 | 221 | 158 | 97 |
| | 0.003 | 141 | 249 | 114 | 169 |

The SC-extract has distinctly stimulated the rate of DNA, ATP, proteins of human fibroblasts cultured in vitro.

### Example 4: Inhibition test on lipoxygenase

Lipoxygenase was incubated with a specific substrate (unsaturated fatty acid) and the product to be tested. Then the rate of released superoxide anions was evaluated by luminol luminescent probes.

The percentage of inhibition versus control of the superoxide anions by the SC-extract at 0.03 % (w/v) was 89 %. This extract has induced a distinct potential to stabilize reactive oxygen species such as superoxide anions generated through lipoxygenase activity.

## Claims

1. The use of the extract of the fruit of Schisandra chinensis for the cosmetic treatment of the human body, wherein this use is directed to an anti-ageing effect, and wherein the extract is obtained by a process comprising
a) extracting of the fruit of Schisandra chinensis with supercritical carbon dioxide to obtain a mixture comprising the extract and the supercritical carbon dioxide
b) removing the supercritical carbon dioxide from the mixture thus obtained.

2. The use according to claim 1, wherein the extract is used in the form of a composition comprising
a) the extract and
b) auxiliaries and/or additives which are common for cosmetic purposes.

3. The use according to claim 2, wherein b) is selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

## Patentansprüche

1. Verwendung des Extrakts aus der Frucht von Schisandra chinensis für die kosmetische Behandlung des menschlichen Körpers, wobei diese Verwendung auf eine Wirkung gegen das Altern gerichtet ist und wobei der Extrakt durch ein Verfahren erhalten wird, das Folgendes umfasst:
a) Extrahieren der Frucht von Schisandra chinensis mit superkritischem Kohlendioxid, wodurch man eine Mischung erhält, die den Extrakt und das superkritische Kohlendioxid umfasst, und
b) Entfernen des superkritischen Kohlendioxids aus der so erhaltenen Mischung.

2. Verwendung nach Anspruch 1, wobei der Extrakt in Form einer Zusammensetzung verwendet wird, die Folgendes umfasst:
a) den Extrakt und
b) für kosmetische Zwecke übliche Hilfsstoffe und/oder Zusatzstoffe.

3. Verwendung nach Anspruch 2, wobei b) aus der Gruppe Ölkörper, Tenside, Emulgatoren, Fette, Wachse, Perlglanzwachse, Konsistenzgeber, Verdickungsmittel, Überfettungsmittel, Stabilisatoren, Polymere, Silikonverbindungen, Lecithine, Phospholipide, biogene Wirkstoffe, Deodorantien, antimikrobielle Mittel, Antiperspirantien, Filmbildner, Mittel gegen Schuppen, Quellmittel, Insektenrepellentien, Hydrotropisierungsmittel, Lösungsvermittler, Konservierungsmittel, Parfümöle und Farbstoffe stammt.

## Revendications

1. Utilisation de l'extrait du fruit de Schisandra chinensis pour le traitement cosmétique du corps humain, cette utilisation étant orientée vers un effet anti-vieillissement, et l'extrait étant obtenu par un procédé comprenant
a) l'extraction du fruit de Schisandra chinensis par du dioxyde de carbone supercritique pour obtenir un mélange comprenant l'extrait et le dioxyde de carbone supercritique,
b) l'élimination du dioxyde de carbone supercritique du mélange ainsi obtenu.

2. Utilisation selon la revendication 1, **caractérisée en ce que** l'extrait est utilisé sous forme d'une composition comprenant
a) l'extrait, et
b) des auxiliaires et/ou additifs qui sont courants à des fins cosmétiques.

3. Utilisation selon la revendication 2, **caractérisée en ce que** b) est choisi dans le groupe constitué de corps huileux, de tensioactifs, d'émulsifiants, de graisses, de cire, des cires nacrées, d'agents de texture, d'épaississants, d'agents de surgraissage, de stabilisants, de polymères, de composés siliconés, de lécithines, de phospholipides, d'ingrédients actifs biogéniques, de déodorants, d'agents antimicrobiens, d'anti-transpirants, d'agents filmogènes, d'agents antipelliculaires, d'agents de gonflement, de répulsifs d'insectes, d'hydrotropes, de solubilisants, de conservateurs, d'huiles parfumées et de colorants.
